# EUROPEAN PATENT SPECIFICATION

(11) **EP 1 742 671 B1**
(45) Date of publication and mention of the grant of the patent: **28.05.2014**
(21) Application number: 05742386.5
(22) Date of filing: 06.05.2005
(51) Int. Cl.: A61L 2/03, A23L 3/005, C02F 1/48, C02F 1/461

(54) **ELECTRIC FIELD FLUID TREATMENT CHAMBER**
FLÜSSIGKEITSBEHANDLUNGSKAMMER MIT ELEKTRISCHEM FELD
ENCEINTE DE TRAITEMENT DE FLUIDE A CHAMP ELECTRIQUE

(30) Priority: 07.05.2004 US 568670 P
(43) Date of publication of application: 17.01.2007
(73) Proprietor: UNIVERSITY OF WATERLOO, Waterloo, Ontario N2L 3G1 (CA)
(72) Inventor: JAYARAM, Sheshakamal, H., Waterloo, Ontario N2L 2R7 (CA); RAO, Lolla Kameswara, Bangalore 560012 (IN)
(74) Representative: Jennings, Nigel Robin
(86) International application number: PCT/CA2005/000699
(87) International publication number: WO 2005/107821

(56) References cited:
- CA-A1- 2 459 875
- US-A- 5 048 404
- US-A- 5 662 031
- US-A- 6 110 423

## Description

### FIELD OF THE INVENTION

The present invention relates generally to a treatment chamber for the electric field treatment of fluids and specifically to an electric field fluid treatment chamber for deactivating microorganisms in fluids.

### BACKGROUND OF THE INVENTION

The conventional electric field fluid treatment chamber consists of a fluid chamber having a fluid treatment zone, a set of electrodes disposed within the fluid treatment zone, and a voltage pulse generator connected to the electrodes. The pulse generator applies voltage pulses to the electrodes, thereby causing the electrodes to induce an electric field in the fluid treatment zone. By controlling the intensity of the electric field within the treatment zone, fluid introduced in the treatment zone can be sterilized or pasteurized without adversely affecting desirable characteristics such as flavour, texture, appearance and nutrient values of the fluid.

A conventional parallel plate electrode treatment chamber comprises a pair of planar electrodes, defining a treatment zone between the electrodes. This arrangement is intended to provide a uniform electric field between the plates. However, the conventional parallel plate treatment chamber geometry causes "edge effects", in that the electric field tends to be of a greater intensity at the edges of the electrodes than in the centre of the electrodes. As a result, the effective treatment volume is limited. Further, such high intensity located at the edges may result in arcing through the fluid being treated, which can adversely affect the desirable food characteristics of the fluid and may disrupt the electric field. Accordingly this may consume additional power in order to re-establish the field. Moreover, the high intensity field at the edges cannot be effectively utilized in the treatment process.

The conventional coaxial electrode treatment chamber has a cylindrical or elongated centre electrode disposed within an elongated tubular outer electrode, and a fluid treatment zone disposed coaxially between the inner electrode and the outer electrode. Fluid, such as pumpable foodstuffs, is pumped into one end of the treatment chamber, through the coaxial fluid treatment zone, and out the opposite end of the treatment chamber. At a practical level, it is understood that the coaxial electrode configuration is advantageous over the parallel plate geometry since it provides larger area of electrode surface that is capable of generating an electric field and that has a higher treatment capacity.

Notwithstanding its enhanced treatment capacity, the foregoing coaxial treatment chamber is far from an optimal solution since it suffers from similar deficiencies to the parallel plate geometry. Edge effects lead to an under utilization of the highest field intensity, which translates into energy inefficiency and higher cost.

The conventional coaxial treatment chambers also allow eddy currents to develop at the mouth of the fluid treatment chamber, which in turn limits the maximum flow rate of the chamber. Further, such chambers may allow treated and untreated products to mix, thereby limiting the effectiveness of the treatment chamber. Accordingly, attempts have been made to improve upon the conventional electric field fluid treatment chambers.

For instance, Bushnell (US Patent 5,048,404) describes a treatment chamber comprising an inner cylindrical electrode surrounded by an outer annular electrode. The inner electrode is tapered at each end. However, as the electrodes impart a largely coaxial configuration to the treatment chamber, the maximum flow rate of fluid that can pass through the treatment zone is limited by the minimum allowable electric field intensity. Also, the electrode design may increase turbulence within the fluid, thereby increasing the likelihood of mixing between treated and untreated product.

Qin (US Patent 5,662,031) describes a treatment chamber which comprising an inner cylindrical electrode surrounded by an outer annular electrode. Both the inner and outer electrodes have scalloped electrode surfaces. However, this arrangement may increase the likelihood of eddy currents developing in the portions of the treatment zone where the electrode surfaces are spaced farthest apart, thereby limiting the maximum flow rate of fluid that can pass through the treatment zone. Further, this arrangement increases agitation of the fluid, thereby increasing the likelihood of frothing or bubble production in the fluid. As a result, the design increases the propensity for electrical discharge between the electrodes.

Mittal (U.S. Patent 6,093,432) describes a treatment chamber comprising a housing containing an inner electrode and an outer electrode disposed about the inner electrode, midway along the length of the inner electrode. The inner electrode comprises a metal pipe having a circular transverse cross-section, and the outer electrode comprises an annular disc having a hole in the centre. However, the outer electrode may cause eddy currents to develop in the treatment zone, thereby limiting the maximum flow rate of foodstuffs that can pass through the treatment zone.

Bushnell (U.S. Patent 6,110,423) describes a serial-electrode treatment cell comprising a first cylindrical electrode, a second cylindrical electrode, and an insulator section disposed serially between the first and second electrodes. The insulator section transitions from an inner radius equivalent to that of the first electrode, to a smaller radius section, back to an inner radius equivalent to that of the second electrode. The transition section in conjunction with the smaller radius section concentrates the electric field in the vicinity of the smaller radius section. However, the smaller radius section also limits the maximum flow rate of foodstuffs that can pass through the treatment zone. Further, since the electric flux lines are parallel to the direction of fluid flow through the treatment zone, some portions of the fluid may become over-treated, while other portions of the fluid may become under-treated.

Therefore, there remains a need for a continuous-flow fluid treatment chamber that treats all portions of fluid in the treatment zone substantially equally. There is also a need for a continuous-flow fluid treatment chamber that is not prone to significant mixing of treated and untreated product.

### SUMMARY OF THE INVENTION

Aspects of the present invention are set out in the appended claims.

### BRIEF DESCRIPTION OF THE DRAWINGS

Embodiments of the invention will now be described, by way of example only, with reference to the drawings, in which:
- **Fig. 1**: is a schematic cross-sectional view of an electrode assembly;
- **Fig. 2**: is a block diagram illustrating a convex annular treatment zone;
- **Fig. 3**: is a schematic cross-sectional view of a batch-mode treatment chamber including the electrode assembly depicted in Fig. 1;
- **Fig. 4a**: is cross-sectional view of a continuous-flow treatment chamber for treating high viscosity fluids;
- **Fig. 4b**: is cross-sectional view of an alternate continuous-flow treatment chamber for treating high viscosity fluids;
- **Fig. 5**: is a schematic vertical cross-sectional view of a continuous-flow treatment chamber for treating low viscosity fluids;
- **Fig. 6**: is a schematic horizontal cross-sectional view of the treatment chamber depicted in Fig. 5;
- **Fig. 7**: is a perspective view of the electrode assembly used in the treatment chamber depicted in Figs. 5 and 6;
- **Fig. 8**: is a schematic cross-sectional view of an alternate electrode assembly to that shown in Fig. 1; and
- **Fig. 9**: is a schematic cross-sectional view of an alternate electrode assembly to that shown in Figs. 1 and 8.

### DETAILED DESCRIPTION OF THE PREFERRED EMBODIMENTS

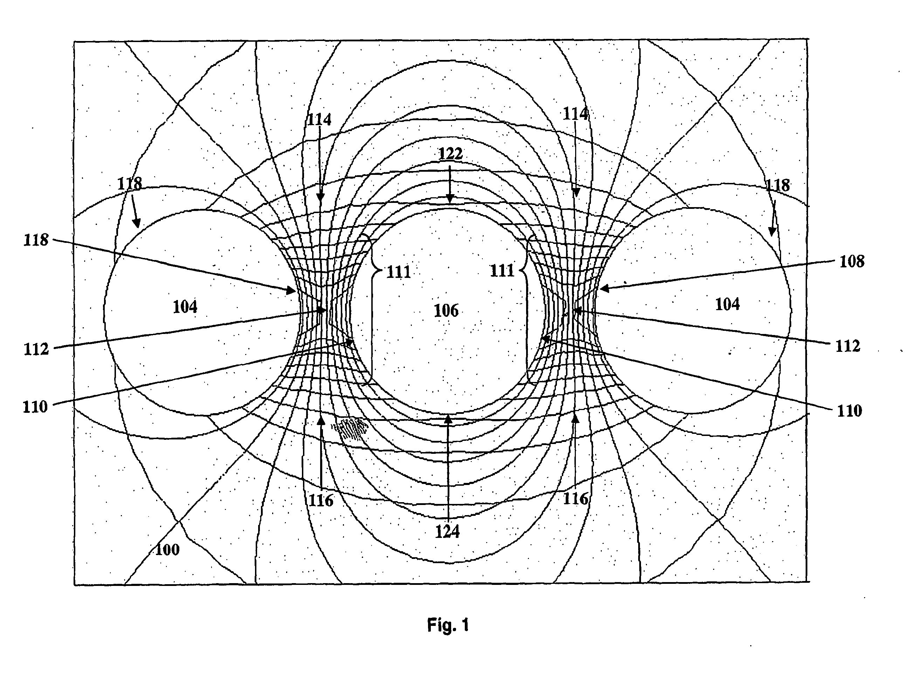

For convenience, like numerals in the description refer to like structures in the drawings. Referring to Fig. 1, a schematic cross-sectional view of an electrode assembly depicting electrode surfaces, a treatment zone, equipotential lines, and electric field contours associated with the electrode surfaces is represented generally by numeral 100. The electrode assembly 100 comprises an outer electrode 104 having an exterior electrode surface 108 and an inner electrode 106 having an exterior electrode surface 110.

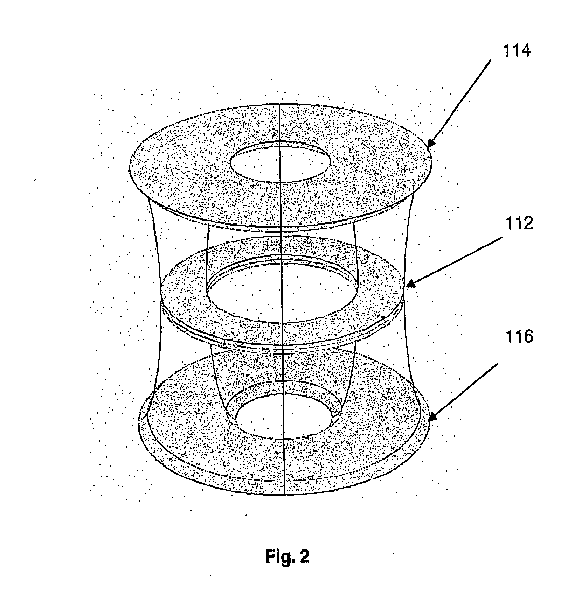

The outer electrode 104 is disposed around the inner electrode 106. The exterior electrode surface 108 of the outer electrode 104 opposes the exterior electrode surface 110 of the inner electrode 106. The opposing electrode surfaces 108 and 110 together define an biconcave annular space that constitutes a treatment zone 111 for treatment of fluid there between. The annular space constituting the treatment zone is described as biconcave since it is defined by two opposing convex surfaces which impart the corresponding concave curvatures to the opposing vertical outline of the annular space in such a manner that the width of the space narrows towards the vertical mid section. Referring to Fig. 2, a perspective view of the annular space created by the opposing electrode surfaces 108 and 110 is shown.

Referring once again to Fig. 1, the opposing electrode surfaces 108 and 110 of the outer and inner electrodes 104 and 106 have respective electrical contacts for connection to a voltage pulse generator (not shown). The voltage pulse generator applies voltage pulses to the opposing surfaces of electrodes 104 and 106, thereby establishing an electric field that is uniform per cross sectional unit plane between the opposing electrode surfaces 108 and 110. Further, the most intense electric field that the electrodes 104 and 106 can generate occurs between the opposing electrode surfaces 108 and 110, as desired.

The electric field established between the opposing electrode surfaces 108 and 110 is continuous, extending throughout the annular space. However, the opposing electrode surfaces 108, 110 are curved so that the intensity of the electric field of any unit area of horizontal cross sectional plane in the annular space depends on the surface curvature and distance between the opposing electrode surfaces 108, 110 at that cross section.

Accordingly, the treatment zone 111 can be divided into three major sections that are characterized by their primary functionality with respect to the treatment process. These sections include a primary treatment zone 112, a treatment zone inlet 114 (hereafter referred to as zone inlet 114) disposed at one end of the primary treatment zone 112, and a treatment zone outlet 116 (hereafter referred to as zone outlet 116) disposed at the opposite end of the primary treatment zone 112. As will be explained, fluid is introduced into the primary treatment zone 112 via the zone inlet 114, and is retrieved from the primary treatment zone 112 through the zone outlet 116. The fluid introduced into the treatment zone 111 is exposed to the electric field generated between the electrode surfaces 108, 110. As a result, the electrode assembly 100 is able to deactivate microorganisms contained in the fluid before the fluid is retrieved from the treatment zone 111.

The opposing electrode surfaces 108, 110 are configured to maintain the electric field distribution continuous through out the treatment zone 111, generate an electric field that is substantially uniform per unit horizontal cross section plane between opposing electrode surfaces 108, 110 in the zone inlet 114 and zone outlet 116, and maintain an electric field distribution of the highest intensity generated by the opposing surfaces substantially uniform within the treatment zone 112. Substantially uniform as defined herein relates to having approximately the same electric field intensity. Accordingly, the opposing electrode surfaces 108, 110 that proximate the annular space constituting the treatment zone 111 are smooth continuous, devoid of edges and substantially convex throughout the treatment zone 111. In particular, the electrode surface 110 of the inner electrode 106 comprises a convex electrode surface, and the electrode surface 108 of the outer electrode 104 comprises a surface of revolution disposed around the inner electrode 106. The opposing electrode surfaces 108, 110 can assume various degrees of convexity (convex curvature) thereby imparting the corresponding various degrees of concavity to the annular space confined by the opposing electrode surfaces.

With the configuration described above, the electric field is continuous with the intensity increasing smoothly moving from the zone inlet 114 towards the primary treatment zone 112, where the electric field is most intense, and then decreasing smoothly moving towards the zone outlet 116. The electric field distribution between the opposing electrode surfaces 108, 110 is such that the intensity is substantially uniform per-unit cross-section area of the treatment inlet and outlet zones 114, 116, and substantially uniform throughout the primary treatment zone 112. Additionally, such a configuration supports a steady and uniform fluid flow that is free of turbulence in the biconcave annular space defined between the opposing electrode surfaces 108 and 110. The fluid flow is guided by the the inner electrode surface 110 such that it circumfuses the surface as it moves from the inlet zone 114 to the outlet zone 116.

Accordingly, it will be appreciated that the treatment zone 111 comprises a substantially biconcave annular space defined by two surfaces that approximate a convex surface configuration. In the present embodiment, the outer electrode's surface 108 bordering the biconcave annular space comprises a toroidal electrode surface and the inner electrode's surface 110 bordering the biconcave annular space comprises a substantially ellipsoidal electrode surface. Also, the biconcave annular space has a vertical orientation, with the zone inlet 114 disposed at an upper section of the space and the zone outlet 116 is disposed at the lower section of the space.

Further, although, as described above, the outer electrode's surface 108 proximate the treatment zone 111 comprises a toroidal electrode surface and the inner electrodes' surface 110 proximate the treatment zone 111 comprise a substantially spherical surface, it will be appreciated that the surfaces of the outer and inner electrodes 104, 106 remote from the treatment zone 111 denoted by numerals 118, 122 and 124 respectively could adopt other shapes, including linear and parabolic, as required by the design details of the fluid treatment application. Moreover, the remote surfaces 118, 122, 124 could be fabricated of non-conducting material as may be suitable to the application.

As such, although the outer electrode 104 in the present embodiment is described as being toroidal in shape and the inner electrode 106 substantially spherical in shape, the overall shape of the electrodes are not limited as such. Rather, other shapes configurations may be used as long as the opposing electrode surfaces 108, 110 proximate the annular space generate and maintain the electric field substantially uniform per unit horizontal cross sectional plane between the opposing electrode surfaces and that such an electric field includes the most intense electric field generated by the opposing electrode surfaces.

In operation, the voltage pulse generator applies a series of voltage pulses to the inner and outer electrodes 104, 106, thereby establishing an electric field in the annular space between the opposing electrode surfaces 108, 110. Untreated fluid, such as water or a liquid foodstuff, is introduced into the primary treatment zone 112 of the annular space. The electric field generated between the opposing electrode surfaces 108, 110 causes electroporation or dielectric rupture of cell membranes, resulting in the inactivation of microorganisms in the fluid. This process is also referred to as cold pasteurization. The cold pasteurization occurs within the treatment zone 111, and particularly within the primary treatment zone 112.

As a result, the treated fluid that is retrieved from the treatment zone 112 at the end of the treatment process is cold pasteurized to a level where it is considered to be safe and acceptable. The treated fluid is packaged and stored under sterile or refrigerated conditions for use. Thus, by properly controlling the intensity of the electric field through the voltage pulses applied by the voltage pulse generator to the opposing electrode surfaces 108, 110, thus to the fluid flowing within the treatment zone 111, the electrode assembly 100 is able to non- thermally sterilize or pasteurize the untreated fluid.

Numerous advantages are realized by using the electrode assembly 100. As discussed above, the opposing electrode surfaces 108, 110 are substantially convex throughout the treatment zone 111. Accordingly, they are devoid of sharp edges, which would cause edge effects to be induced in the electric field within the treatment zone 111. Further, the distribution of the electric field is continuous. Thus, the intensity of the electric field increases smoothly from the zone inlet 114 towards the midsection of the primary treatment zone 112, and then decreases smoothly again towards the zone outlet 116.

Also, the intensity of the electric field is substantially uniform per unit horizontal cross-section plane of the zone inlet 114 and zone outlet 116. Within the primary treatment 112, where the most intense distribution of the electric field generated is located, and the electric field is substantially uniform with approximately the same intensity. With this configuration, and under steady and uniform fluid flow conditions, all portions of the fluid moving through the primary treatment zone 112 are treated with substantially the same intensity of electric field, thereby limiting the extent to which fluid may be over treated or under treated.

Further, the electrode assembly 100 described above generates and maintains (under conditions of substantially steady and uniform fluid flow) the most intense electric field produced by the opposing electrode surfaces 108, 110 within the primary treatment zone 112 for utilization in the treatment process. This is contrast to the current state of the art electric field treatment chamber in which the most intense field is located on edges and is under utilized. As a result, the dosage, and particularly the dosage within the primary treatment zone 112, is higher than that which can be obtained with the prior art. The term "dosage" is defined herein as a unit volume of fluid exposed to electric field at a predetermined flow rate, with respect to the electric field intensity provided by the electrode assembly 100. Accordingly, a cost savings may be realized in terms of the energy required for operation of the chamber 100, as compared to the prior art.

Still further, the electrode assembly 100 can influence the fluid dynamics of the fluid flow within the treatment zone 111. The smooth and convex surface of the opposing electrode surfaces 108, 110 support a flow in which turbulence is significantly reduced to the extent that the flow is steady and uniform. Unlike the current state of the art electric field chambers in which fluid is forced under pressure through the treatment zone, the flow in the treatment zone of the present electrode assemble is under the influence of gravity and atmospheric pressure with the fluid overflowing and circumfusing surface 110 of the inner electrode that guides the fluid flow to the primary treatment zone 112

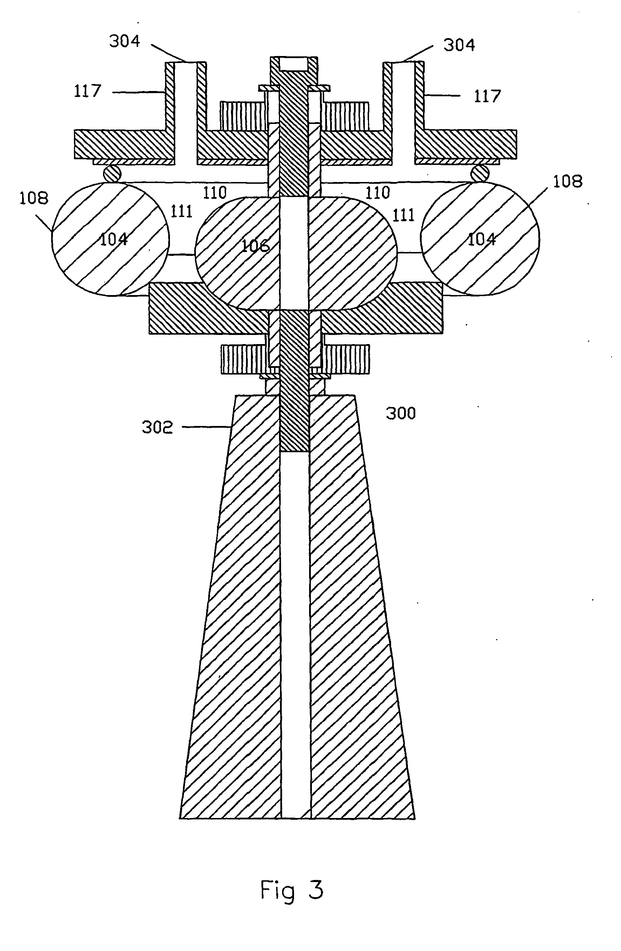

Referring to Fig. 3, a treatment chamber for batch mode water treatment is illustrated generally by numeral 300. In the present embodiment, the treatment chamber 300 comprises a support 302 for supporting the electrode assembly 100, a channel 304 for receiving treated water, and an annular conduit 306 for interfacing between the electrode assembly 100 and a fluid to be treated. In operation, a discrete sample of untreated fluid is introduced into the zone inlet 114 as an annular curtain via the annular conduit 306 disposed above the treatment zone 111. The fluid is treated as it passes through the treatment zone 111 and exits via the zone outlet 116 into the channel 304. The fluid can then be retrieved from the reservoir 304 and is handled and stored as required. This embodiment is well suited for treating batches of viscous fluid. As will be appreciated, the present embodiment can be made continuous by providing a continuous source of fluid and a means of continuous retrieval.

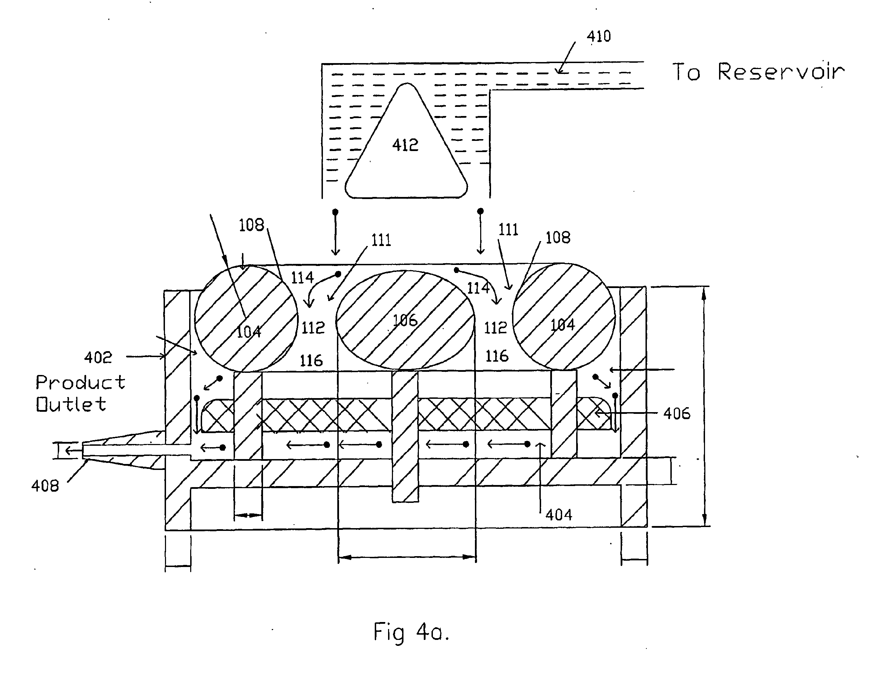

Referring to Fig. 4a, a treatment chamber for continuous flow water treatment is illustrated generally by numeral 400. In the present embodiment, untreated fluid is continuously introduced into the treatment zone 111 and retrieved continuously from the treatment zone 111. The treatment chamber 400 comprises a support 402 for supporting the electrode assembly 100 and a channel 404 for retrieving the treated water. A disc 406 is provided between the electrode assembly 100 and the channel 404. An outlet 408 is provided to facilitate removal of the treated fluid from treatment chamber. A delivery tube 410, including a baffle 412, is provided for facilitating input of the fluid into the treatment chamber 400.

In operation, untreated fluid from a reservoir (not shown) above the treatment chamber 400 is introduced into the treatment zone 111 via a delivery tube 410 disposed above the zone inlet 114. The baffle 412 in the delivery tube 410 directs the fluid into the shape of an annular curtain for passage through the treatment zone 111. In addition, the flow rate of the untreated fluid exiting the tube 410 can be controlled by valves or other known flow control mechanisms (not shown) to provide a desired rate of flow to the inlet zone 114. Treated fluid exits the treatment zone and is dispersed towards an outer edge of the disc 406 and into the channel 404. The fluid then flows out of the treatment chamber via the outlet 408.

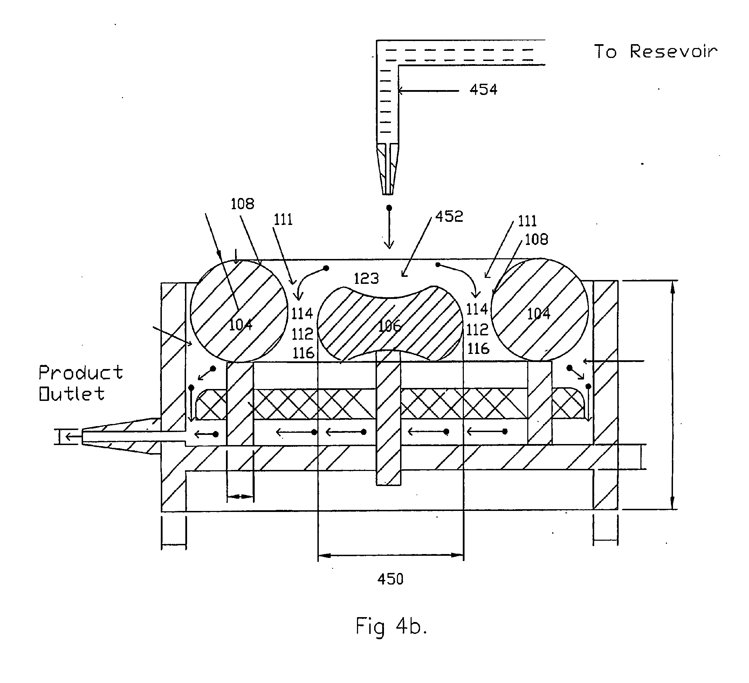

Referring to Fig. 4b, an alternate embodiment of a treatment chamber for continuous flow water treatment to that shown in Fig. 4a is illustrated generally by numeral 450. In the present embodiment, the upper surface of the second electrode 106 includes a depression 452. Further, a delivery tube 454 is disposed above the depression 452 and arranged to deliver the fluid therein.

In operation, untreated fluid exiting the delivery tube 454 fills the depression 452, which overflows radially outwards circumfusing the electrode surface 110 of the inner electrode 106 and is further guided by the electrode surface 110 through the zone inlet 114 to the primary treatment zone 112.

Both of these latter embodiments are also well suited for, but need not be limited to, the continuous treatment of viscous fluids, including fluid with fine particulate matter.

The electrode assembly 100 and configurations described herein are well suited for a steady and uniform continuous-flow mode operation since the opposing electrode surfaces 108, 110 are smooth and continuous without edges and corners and thereby minimizing, if not eliminating, the likelihood of fluid stagnation and eddy currents in the entire treatment zone 111. Furthermore such configurations reduce the likelihood that untreated fluid will become mixed with treated fluid. As a result, the effectiveness of treatment for each unit volume within the treatment zone 111 is consistent and predictable, leading to an overall all effective treatment process. This is particularly true for industrial operations where outcome predictability is highly desirable. Also, the fluid flow is guided by the curvature of the opposing electrode surfaces 108, 110, and in particular by the electrode surface 110 of the inner electrode 106 through the treatment zone 111 under influence of gravity alone. As a result, fluid turbulence becomes insignificant and the occurrence of eddy currents being induced in the fluid flow in the treatment zone 111 is limited further.

Additionally, the opposing electrode surfaces 108, 110 provide a substantially steady, uniform and axisymmetric flow of the fluid over the electrode surface 110 and through the treatment zone 111. The flow is considered steady and substantially uniform since under the influence of gravity the velocity, pressure and density are substantially constant with time and the velocity vector is substantially identical in magnitude and direction, particularly in the primary treatment zone 112 where the fluid is exposed to the strongest electric field. As a result, all portions of the fluid have substantially the same flow rate and the same residence time through the treatment zone 111.

Since the electric field induced is substantially uniform per unit cross-section of the treatment zone 111, all portions of the fluid flowing through a particular cross-section area of the treatment zone 111 are exposed to substantially the same intensity of electric field per unit cross-sectional area. Accordingly, the dosage of electric field applied to the fluid within the entire treatment zone can be readily controlled through the energy of the voltage pulse applied to the electrodes and can also be readily predetermined. With a substantially steady and uniform flow, the probability of induced breakdown of the electric field due to turbulence and bubble formation is reduced and once established between the opposing electrode surfaces 108, 110, the electric field is maintained throughout the treatment process. Maintaining the consistency in the electric field throughout the treatment process contributes to the accuracy and confidence limit of the dosage experienced by the fluid undergoing treatment under preset operating conditions of applied voltage and fluid flow. From an economic perspective, the reduction of turbulence and bubble formation also contributes energy savings in the operation.

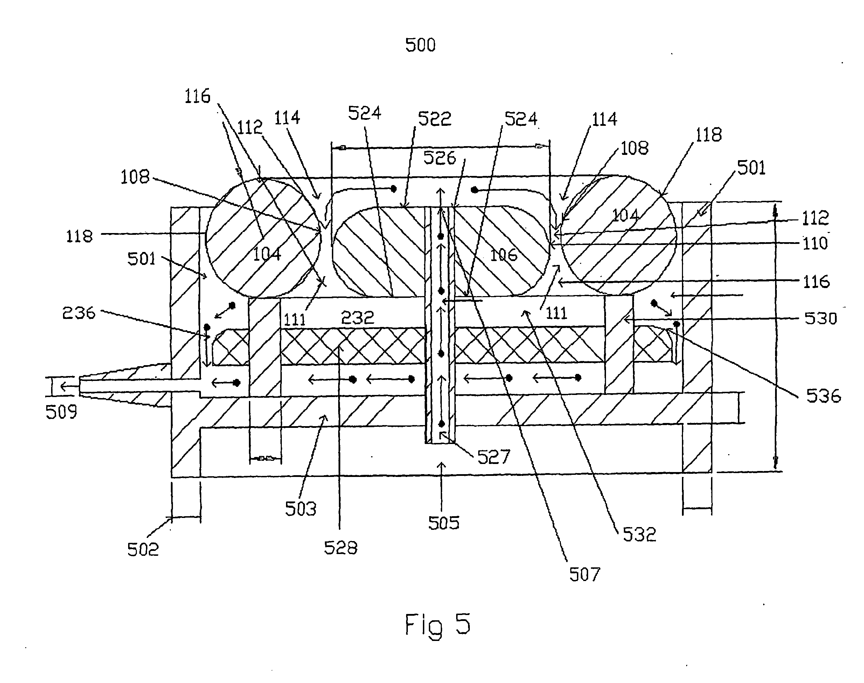

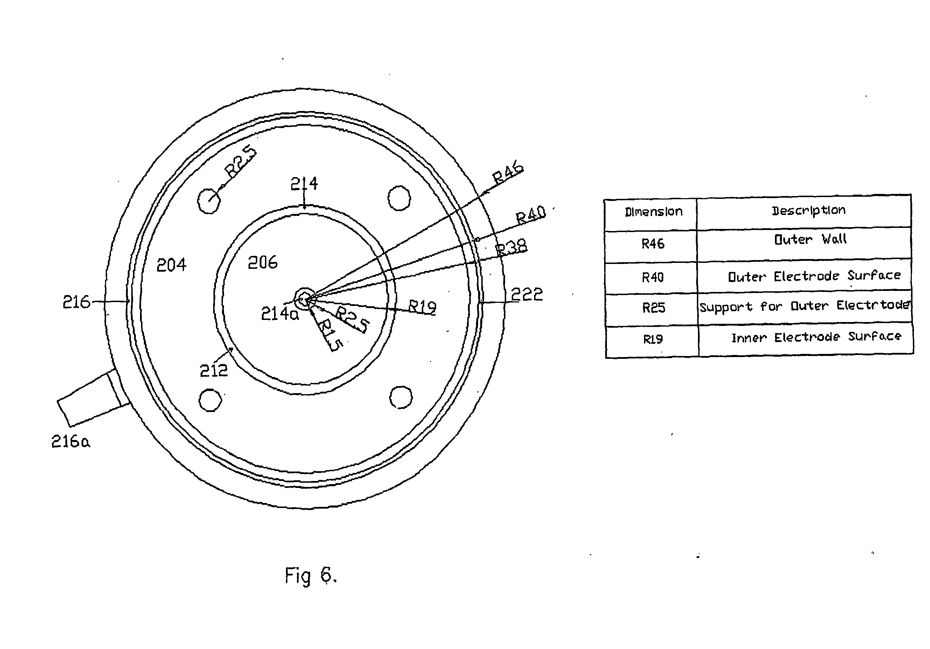

Referring to Fig. 5 a vertical cross-sectional view of a treatment chamber in accordance with yet an alternate embodiment of the present invention is illustrated generally by numeral 500. Further, referring to Fig 6 a horizontal cross sectional view of the treatment chamber shown in Fig. 5 is illustrated generally by numeral 600.

The treatment chamber 500 comprises a housing 502 and an electrode assembly 100 disposed therein. The housing 502 comprises a substantially vertical sidewall 501, and a substantially planar base 503 supporting the sidewall 501. Together, the sidewall 501 and the planar base 503 comprise a fluid-tight container. As shown in Figs. 5 and 6, the sidewall 501 is disposed around the perimeter of the planar base 503, and encloses the electrode assembly 100 therein. The planar base 503 includes a fluid inlet port 505 for introducing untreated fluid through the housing 502 and into the chamber 500, as will be described. The sidewall 501 includes a fluid outlet port 509 for collecting treated fluid from the treatment chamber 500.

The housing 502 includes, within its interior, electrically insulated electrode mounting frames for supporting the electrode assembly 100. The mounting frames for the electrodes are disposed inside the housing 502, but outside the treatment zone 111. This arrangement reduces the likelihood of eddy currents forming in the treatment zone 111 and maintains the uniformity of the electric field throughout the treatment zone 111.

As shown in Figs. 5 and 6, the outer electrode 104 is substantially toroidal in shape, and the inner electrode 106 is substantially spherical in shape. In the present embodiment, the electrode surface of the inner electrode 106 includes a substantially spherical outer electrode surface 108, a substantially planar upper electrode surface 522, and a substantially planar lower electrode surface 524. The electrode surface of the toroidal outer electrode 104 circumscribes the spherical opposing electrode surface 110.

The inner electrode 106 also includes a substantially vertical fluid bore 526 extending through the centre of the inner electrode 106, between the upper and lower planar electrode surfaces 522, 524. The upper end of the fluid bore 526 terminates at the upper electrode surface 522 and is in communication with a radially dispersion zone 534 there above. The lower end of the fluid bore 526 terminates at the lower electrode surface 524.

It will be appreciated that although, in the present embodiment, the sidewall 501 is substantially cylindrical and the base 503 is substantially circular, the housing 502 could have an alternate shape to accommodate alternate designs. As previously described, the shape of the electrode assembly 100 may differ in accordance with the shapes of the surfaces 122, 124 and 118 of the inner and outer electrodes that are remote from the treatment zone.

The electrode mounting frame secures the electrodes 104, 106 in position within the housing 502 and maintains a fixed separation between the opposing electrode surfaces 108 and 110. Consequently, a constant dimension and configuration of the annular space defined by the opposing surfaces is retained.

The housing 502 further comprises a substantially planar circular plate 528, and a plurality of support posts 530 secured at their respective lower ends to the base 503. The support posts 530 are disposed adjacent to the perimeter of the circular plate 528, and extend vertically upwards through the circular plate 528.

The fluid inlet port 505 extends through the base 503 and the circular plate 528, and communicates with the lower end of a fluid channel 527. The fluid channel 527 extends through the bore 526 in the inner electrode 104. Although the fluid channel is shown as being cylindrical, the fluid channel need not be limited to a cylindrical channel but may assume any geometrical hollow space inside the inner electrode e.g. preferably spherical or elliptical where the fluid temporary reside in order to dampen pulsing effect of the fluid flow before emerging from the output port 507 located on the upper surface 522 of the inner electrode 106. The fluid inlet port 505 is coupled to an external pump (not shown) for supplying the fluid at a desirable rate into the fluid channel 527. The fluid fills the channel 527 before emerging from the output port 507 and dispersing radially outwards in a steady and uniform manner over the upper surface 522 and into the zone inlet 114.

The support posts 530 are secured at their respective upper ends to the base of the first electrode 104. The support posts 530 are separated from one other to allow treated fluid exiting the outlet zone 116 to fall into a discharge zone 532 and flow outwards along the upper surface of the circular plate 528 towards its perimeter. An annular discharge channel 534 is maintained between the outer edge of the circular plate 528 and the inner surface of the side wall 501 so that treated fluid travelling outwards along the circular plate 228 can flow downwards through the annular discharge channel 536 and exit the housing 502 via the fluid exit port 209.

The arrows in Fig. 5 represent the movement of fluid through the fluid treatment chamber 500. As shown, untreated fluid is introduced into the fluid inlet port 505 and moved upwards through the fluid channel 527. When the fluid channel 527 is filled, untreated fluid exits the fluid bore 526, overflowing radially from the fluid output port 507 and onto the upper electrode surface 522. The overflowing fluid, guided by the curvature of the upper electrode surface 522 disperses radially outwards circumfusing electrode surfaces 522 and 110 to flow in a substantially uniform manner into the zone inlet 114 and through the primary treatment zone 112.

Accordingly, it can be seen that the fluid passes through the primary treatment zone 112 under influence of gravity alone. To maintain uniformity of fluid flow through the treatment zone 111, force at which the untreated fluid is introduced into the fluid channel 527 is selected such that the untreated fluid rises steadily and substantially uniformly through the fluid channel 527. This minimizes turbulence, pulse and ripple effects, and maximizes radial uniformity on the upper surface 522 when the fluid exits the output port 507.

The untreated fluid is treated by exposure to the electric field in the treatment zone 111, in particular the primary treatment zone 112. The treated fluid exits the treatment zone 112 at the zone outlet 116, flows onto the circular plate 528 of the mounting frame. The zone outlet 116 is disposed at a sufficient distance above the circular plate 528 to allow the untreated fluid to flow freely through the primary treatment zone 112, without mixing with the treated fluid as it exits the zone outlet 116. The fluid moves laterally outwards in all directions over the circular plate 528, past the support posts 230, downwards through the annular discharge channel 536, and exits the treatment chamber 500 through the fluid outlet port 509.

Since the untreated fluid, guided by the smooth and continuous surface of the inner electrode 106, is introduced into the treatment zone 111 in a substantially steady and uniform manner and the flow through the treatment zone 111 is only acted upon by the influence of gravity, the fluid flow remains substantially steady uniform and axisymmetric with respect to the curvature of the inner electrode 106. With such a flow, turbulence in the fluid is either not present or does not result in a significant disruption in the distribution of the electric field. Accordingly, arcing and eddy currents induced in the treatment zone 111, and particularly the primary treatment zone 112 where the most intense electric field is utilized, are either eliminated or significantly reduced.

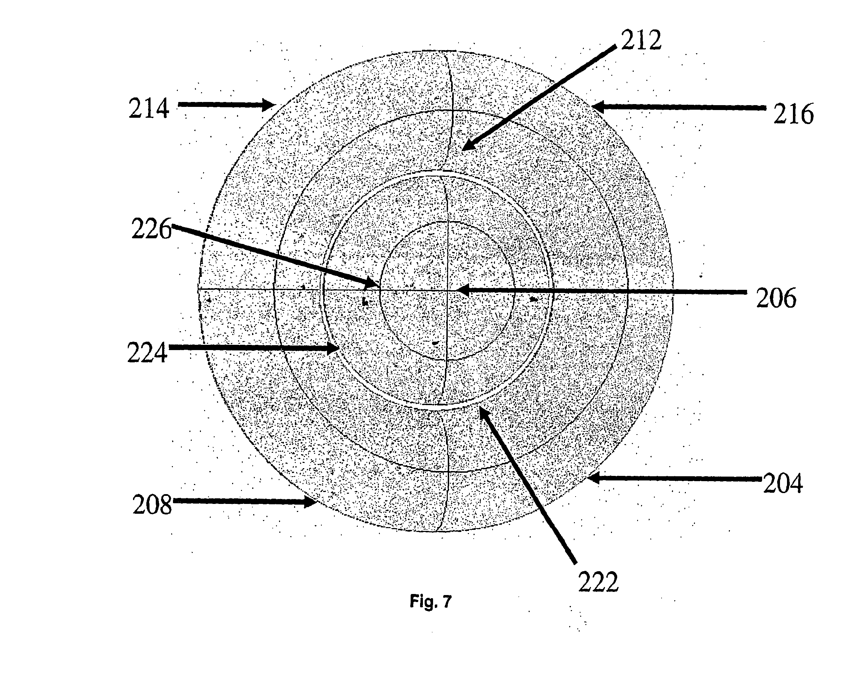

Referring to Figs. 7-9, several embodiments of the electrode assembly 100 are illustrated. Referring to Fig. 7, a perspective view of an electrode assembly 100 comprising a substantially inner spherical electrode and an outer toroidal electrode is shown.

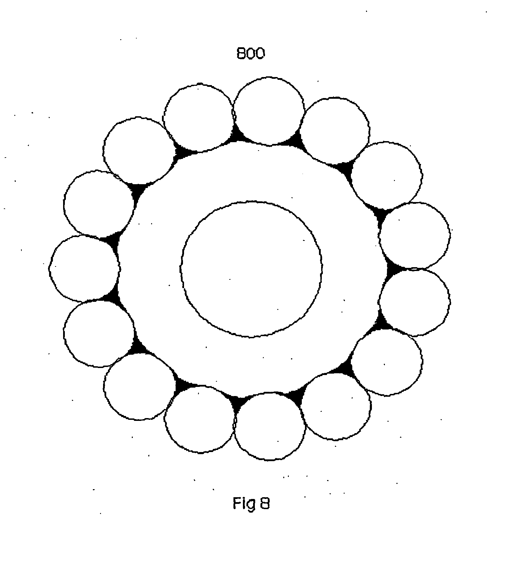

Referring to Fig 8, a horizontal cross sectional view of an electrode assembly 100 comprising a single substantially spherical inner electrode centred within the space created by a ring or a circle of substantially spherical outer electrodes. Between the adjacent opposing surfaces of the outer electrodes in the ring there is affixed either a conducting or a non-conducting fillet. The fillet prevents or inhibits treated fluid from lodging in an intersphere crevice. Further, conducting fillets may be additionally employed to ensure the electric field is sufficiently uniform.

Generally, features such as an electrical field substantially uniform in unit area of a horizontal cross sectional plane; utilization of the most intense field generated by the opposing surfaces defining an annular space; and support of a steady and substantially uniform fluid flow to reduce turbulence, arcing and eddy currents are attainable by an electrode assembly comprising two or more adjacent electrodes having substantially convex adjacent the annular space.

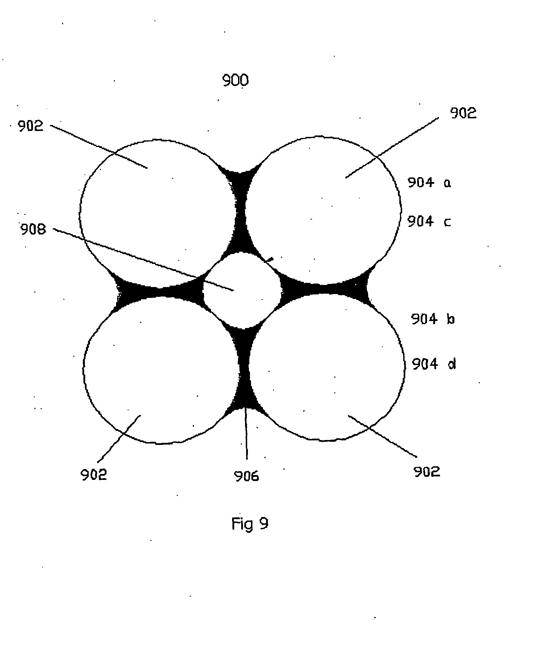

For example, referring to Fig 9, an alternate electrode assembly is shown generally by numeral 900. The electrode assembly 900 comprises of four electrodes 902. The electrodes 902 are substantially spherical and opposing surfaces 904 are determined not from adjacent electrodes but rather between diagonally opposing electrodes in the assembly. For example, 904a and 904c are considered opposing surfaces and 904b and 904d are considered opposing surfaces. A non-conductive fillet 906 is positioned between adjacent electrodes to act as a separator of the electrodes and also to maintain an annular space 908. In such an assembly the fluid flow to the treatment zone is similar to the embodiments described with reference to Figs. 4a and 4b. Alternately, the fluid flow could be similar to the embodiment described with reference to Fig 5.

An electrode assembly having two or three electrodes can also produce a space with the features described above between the adjacent electrodes. However, practically the preferred configuration is an annular space defined by two opposing surface of an electrode assembly, the electrode assembly having an inner electrode and an outer electrode disposed there about.

Referring to Table 1 below, the average microbial decay obtained in the treatment of unpasteurized apple juice with a conventional parallel plate electrode treatment chamber versus an electrode assembly 100 in accordance with the present invention is shown.

**Table 1**

| Chamber type | Process conditions | Average microbial decay, log reduction | Process conditions | Average microbial decay, log reduction |
|---|---|---|---|---|
| Parallel plate (conventional) | 46°C, 90kV/cm, 40 pulses | 1.73 | 50°C, 90kV/cm, 40 pulses | 1.77 |
| Electrode Assembly 100 | 46°C, 80kV/cm, 9 pulses | 2.00 | 50°C, 80kV/cm, 9 pulses | 2.80 |

Due to constraints of the parallel plate chamber, the process conditions are not identical. However, they are substantially similar to demonstrate the advantages of the subject treatment chamber over the prior art. The electric field and the number of pulses applied to the parallel plate chamber were 90kV/cm and 40 pulses respectively compared with 80kV/cm and 9 pulses applied to the subject chamber. The applied pulses were identical in amplitude and frequency and originated from the same pulse generator. Additionally, the parallel plate chamber was operated under batch mode, where the duration of exposure to the electric field was longer than that of the subject chamber, which was operated under continuous mode flow operation of 5ml/min.

In-spite of the higher field, greater number of pulses and exposure time to the parallel plate chamber, the subject chamber was shown to be superior in its performance at temperatures of 46°C and 50°C. The performance was more significant at the higher temperature where the microbial reduction was about 59% compared to 16% at 46°C. These results demonstrate that the electrode assembly 100 is more effective treating fluid foodstuffs than the state of the art treatment chamber. Further, this increased effectiveness is achieved with lower energy consumption than the state of the art treatment chamber.

In accordance with yet a further embodiment, the fluid is pre-treated prior to passage through the treatment chamber. The fluid may be pre-treated by subjecting the fluid to temperature adjustment to a temperature of 40 to 60°C, preferably 50 to 55°C , before exposing the fluid to the electric field. The fluid may be heated by conventional means to the desired temperature range before being introduced into the fluid channel. Alternately, the fluid may be subjected to a blanket of infrared radiation of suitable intensity by focusing the radiation onto the fluid as it emerges from the fluid channel. In this manner, the fluid absorbs the infrared radiation and increases in temperature to the desired level. It will be understood that the pre-heat treatment of some fluids exaggerates the microbiological membrane damage during the electric field treatment process.

Referring to Table 2 below, the average E-coli count obtained by treating contaminated water using the electrode assembly 100 in a variety of test conditions under the continuous flow mode of operation is shown.

**Table 2**

| Grab 1 | Grab 2 | Grab 3 |
|---|---|---|
| Sample description: De-ionized water with E-Coli samples. Tested at room temperature without IR | Sample description: De-ionized water with E-Coli samples. Untreated, but exposed | Sample description: De-ionized water with E-Coli samples. Tested under pre-treatment |
| light. Voltage 20 kV pulse | to IR light. | conditions with IR light Temperature was 36 to 40°C, and 20kV pulse. |
| Count for E-Coli:=> 783000000 | 852000000 | 640000* |
| Count for Total Coliforms:=>886000000 | 901000000 | 2280000 |

These results demonstrate the effectiveness of the electrode assembly 100 in treating fluids, which in this example is water, when the fluids are subjected to a pre-treatment involving temperature adjustment. In the present example, the pre-treatment is an irradiation of the fluids with infrared light. The addition of a pre-treatment stage has better results than treatment using the electrode assembly 100 alone or treatment using infrared light alone.

The present invention is defined by the claims appended hereto. The foregoing description is illustrative of preferred embodiments of the present invention. Those of ordinary skill may envisage certain modifications to the claimed invention which, although not explicitly described or suggested herein, do not depart from the scope of the invention, as defined by the appended claims.

## Claims

1. A treatment chamber (500) for deactivating microorganisms in a fluid, the treatment chamber comprising:
a housing (502) comprising a fluid inlet (505) for receiving fluid to be treated and a fluid outlet (509) for allowing treated fluid to be retrieved; and
an electrode assembly (100) within the housing,
**characterised in that**
the electrode assembly (100) comprises an inner electrode (106) and an outer electrode (104) circumscribing the inner electrode; opposing surfaces of the inner electrode (106) and outer electrode (104) define a biconcave annular treatment zone (111), the opposing surfaces of the inner electrode (106) and outer electrode (104) are convex and continuous throughout the treatment zone.

2. The treatment chamber (500) of claim 1, wherein the electrode assembly (100) is configured to receive a voltage pulse that generates an electric field between the inner electrode (106) and outer electrode (104), wherein the intensity of the electric field decreases in a smooth continuous decrease in intensity of electric field in either direction away from a mid section of the treatment zone (111) when the voltage pulse is applied to the electrodes (104, 106).

3. The treatment chamber (500) of claim 1 or 2, wherein the convex surface of the outer electrode (104) is substantially toroidal and the convex section of the inner electrode (106) is substantially ellipsoidal.

4. The treatment chamber (500) of claim 3, wherein the convex surface of the inner electrode (106) is substantially spherical.

5. The treatment chamber (500) of claim 1 or 2, wherein the convex surface of the outer electrode (104) comprises a plurality of adjacent substantially ellipsoidal surfaces and the convex surface of the inner electrode (106) is substantially ellipsoidal.

6. The treatment chamber (500) of claim 5, wherein the convex surface of the inner electrode (106) is substantially spherical.

7. The fluid treatment chamber of any preceding claim, wherein the treatment zone (111) comprises a zone inlet (114) for receiving untreated fluid, a zone outlet (116) for dispensing treated fluid, and a primary treatment zone (112) for treating the untreated fluid, the primary treatment zone (112) being located in the midsection of the treatment zone (111), between the zone inlet (114) and the zone outlet (116).

8. The treatment chamber (500) of claim 7, wherein a top surface of the inner electrode (106) receives the fluid from a fluid source and conveys it radially by overflow to circumfuse the surface of the inner electrode to introduce the fluid into the zone inlet (114) and the primary treatment zone (112).

9. The fluid treatment chamber (500) of claim 8, wherein the intensity of the electric field gradually increases from the zone inlet (114) towards the primary treatment zone (112) and then decreases gradually from the primary treatment zone (112) towards the zone outlet (116).

10. The fluid treatment chamber (500) of claim 8, wherein the inner electrode (106) includes a fluid bore (526) extending there through along its polar axis, the fluid bore (526) being configured such that the treatment zone (111) is in fluid communication with the fluid inlet.

11. The fluid treatment chamber (500) of claim 10, wherein the inner electrode (106) is substantially planar on its top surface and arranged to facilitate continuous, even and radial communication of the fluid from the fluid bore (526) to the zone inlet (114).

12. The fluid treatment chamber (500) of claim 8, 9, 10 or 11, wherein the inner electrode (106) comprises a depression (452) on its top surface for receiving the fluid from the fluid source.

13. A treatment chamber (500) of any of claims 1 to 12, wherein there is simultaneously produced by the application of a voltage pulse to the inner and outer electrode (104, 106):
a most intense electric field generated by the inner electrode (106) and the outer electrode (104) at a midsection of the biconcave annular space;
a substantially uniform electric field per unit cross section of the biconcave annular space; and
a smooth continuous decrease in intensity of electric field in either direction away from the midsection of the biconcave annular space.

14. The treatment chamber of any of claims 1 to 12, wherein at least one of the opposing surfaces of the inner electrode (106) and outer electrode (104) controls dynamics of flow of the fluid to be treated within the treatment zone.

15. A pasteurization kit for treating a fluid comprising the treatment chamber of any of claims 1 to 14.

16. A method for pasteurizing a fluid **characterised by** the steps of:
generating an electric field between an inner electrode (106) and an outer electrode (104) circumscribing the inner electrode, opposing surfaces of the inner electrode (106) and outer electrode (104) define a biconcave annular treatment zone (111), the opposing surfaces of the inner electrode (106) and outer electrode (104) are convex and continuous throughout the treatment zone (111);
passing the fluid through the biconcave annular treatment zone (111) to expose the fluid to the electric field to inactivate microorganisms in the fluid.

17. The method of claim 16, wherein the outer electrode surface is substantially toroidal and the inner electrode surface is substantially ellipsoidal.

18. The method of claim 16 or 17, wherein the treatment zone (111) comprises a zone inlet for receiving untreated fluid, a zone outlet for retrieving treated fluid and a primary treatment zone (112) for treating the untreated fluid, the primary treatment zone being located in the midsection of the treatment zone between the zone inlet (114) and the zone outlet (116).

19. The method of claim 18, wherein the electric field has its greatest intensity within the primary treatment zone (112).

20. The method of claim 19, wherein the electric field is continuous and substantially uniform per unit cross sectional plane in the zone inlet (114) and zone outlet (116), the intensity of which increases smoothly from the zone inlet (114) towards the primary treatment zone (112) and decreases smoothly from the primary treatment zone (112) to the zone outlet (116).

21. The method of claim 20 further comprising the steps of:
retrieving the fluid from a fluid source and conveying it to the zone inlet (114); and
retrieving treated fluid after it has passed through the zone outlet (116).

22. The method of claim 21, wherein a top surface of the inner electrode (106) receives the fluid from a fluid source and conveys it radially by overflow to circumfuse the surface of the inner electrode (106) in order to introduce the fluid into the zone inlet (114).

23. The method of claim 22, wherein the inner electrode (106) includes a fluid bore extending there through along its polar axis, the fluid bore (526) being configured such that the treatment zone (111) is in fluid communication with the fluid source via a fluid inlet (114).

24. The method of claim 23, wherein the inner electrode (106) is substantially planar on its top surface and facilitates continuous, even and radial communication of the fluid from the fluid bore (526) to the zone inlet (114).

25. The method of claim 22, 23 or 24, wherein the inner electrode (106) comprises a depression (452) on its top surface for receiving the fluid from the fluid source and conveying the received fluid in a smooth, steady and uniform overflow radially toward the zone inlet (114).

26. The method of any of claims 21 to 25 further comprising a pretreatment step, the pretreatment step for adjusting the temperature of the fluid to a predefined level prior to exposing the fluid to the electric field.

27. The method of claim 26, wherein the temperature of the fluid is increased by irradiating the fluid with infrared radiation.

## Patentansprüche

1. Behandlungskammer (500) zum Deaktivieren von Mikroorganismen in einem Fluid, wobei die Behandlungskammer Folgendes umfasst:
ein Gehäuse (502), das einen Fluideinlass (505) zum Aufnehmen von zu behandelndem Fluid und einen Fluidauslass (509), um eine Wiedergewinnung von behandeltem Fluid zuzulassen, aufweist; und
eine Elektrodenanordnung (100) in dem Gehäuse,
**dadurch gekennzeichnet, dass**
die Elektrodenanordnung (100) eine innere Elektrode (106) und eine die innere Elektrode umgebende äußere Elektrode (104) aufweist; gegenüberliegende Oberflächen der inneren Elektrode (106) und der äußeren Elektrode (104) eine bikonkave, ringförmige Behandlungszone (111) definieren und die einander gegenüberliegenden Oberflächen der inneren Elektrode (106) bzw. der äußeren Elektrode (104) in der gesamten Behandlungszone konvex und ununterbrochen sind.

2. Behandlungskammer (500) nach Anspruch 1, wobei die Elektrodenanordnung (100) konfiguriert ist, einen Spannungsimpuls zu empfangen, der zwischen der inneren Elektrode (106) und der äußeren Elektrode (104) ein elektrisches Feld erzeugt, wobei die Stärke des elektrischen Feldes mit einer gleichmäßigen, ununterbrochenen Abnahme der Stärke des elektrischen Feldes in jeder Richtung weg von dem Mittelabschnitt der Behandlungszone (111) abnimmt, wenn der Spannungsimpuls an die Elektroden (104, 106) angelegt wird.

3. Behandlungskammer (500) nach Anspruch 1 oder 2, wobei die konvexe Oberfläche der äußeren Elektrode (104) im Wesentlichen toroidförmig ist und der konvexe Abschnitt der inneren Elektrode (106) im Wesentlichen ellipsoidförmig ist.

4. Behandlungskammer (500) nach Anspruch 3, wobei die konvexe Oberfläche der inneren Elektrode (106) im Wesentlichen kugelförmig ist.

5. Behandlungskammer (500) nach Anspruch 1 oder 2, wobei die konvexe Oberfläche der äußeren Elektrode (104) mehrere benachbarte im Wesentlichen ellipsoidförmige Oberflächen aufweist und die konvexe Oberfläche der inneren Elektrode (106) im Wesentlichen ellipsoidförmig ist.

6. Behandlungskammer (500) nach Anspruch 5, wobei die konvexe Oberfläche der inneren Elektrode (106) im Wesentlichen kugelförmig ist.

7. Fluidbehandlungskammer nach einem vorhergehenden Anspruch, wobei die Behandlungszone (111) einen Zoneneinlass (114) zum Empfangen von unbehandeltem Fluid, einen Zonenauslass (116) zum Ausgeben von behandeltem Fluid und eine primäre Behandlungszone (112) zum Behandeln des unbehandelten Fluids aufweist, wobei sich die primäre Behandlungszone (112) in dem Mittelabschnitt der Behandlungszone (111) zwischen dem Zoneneinlass (114) und dem Zonenauslass (116) befindet.

8. Behandlungskammer (500) nach Anspruch 7, wobei eine obere Oberfläche der inneren Elektrode (106) das Fluid von einer Fluidquelle empfängt und es radial durch Überlaufen transportiert, damit es die Oberfläche der inneren Elektrode umgießt, um das Fluid in den Zoneneinlass (114) und die primäre Behandlungszone (112) einzuleiten.

9. Fluidbehandlungskammer (500) nach Anspruch 8, wobei die Stärke des elektrischen Feldes von dem Zoneneinlass (114) zu der primären Behandlungszone (112) allmählich zunimmt und dann von der primären Behandlungszone (112) zu dem Zonenauslass (116) allmählich abnimmt.

10. Fluidbehandlungskammer (500) nach Anspruch 8, wobei die innere Elektrode (106) eine Fluidbohrung (526) aufweist, die längs ihrer Polarachse durch sie verläuft, wobei die Fluidbohrung (526) in der Weise konfiguriert ist, dass die Behandlungszone (111) in Fluidkommunikation mit dem Fluideinlass steht.

11. Fluidbehandlungskammer (500) nach Anspruch 10, wobei die innere Elektrode (106) auf ihrer oberen Oberfläche im Wesentlichen eben ist und dafür ausgelegt ist, eine ununterbrochene, gleichmäßige und radiale Kommunikation des Fluids von der Fluidbohrung (526) zu dem Zoneneinlass (114) zu erleichtern.

12. Fluidbehandlungskammer (500) nach Anspruch 8, 9, 10 oder 11, wobei die innere Elektrode (106) auf ihrer oberen Oberfläche eine Vertiefung (452) aufweist, um das Fluid von der Fluidquelle zu empfangen.

13. Behandlungskammer (500) nach einem der Ansprüche 1 bis 12, wobei durch das Anlegen eines Spannungsimpulses an die innere und die äußere Elektrode (104, 106) gleichzeitig erzeugt werden:
ein stärkstes elektrisches Feld, das durch die innere Elektrode (106) und die äußere Elektrode (104) in einem Mittelabschnitt des bikonkaven, ringförmigen Raums erzeugt wird;
ein im Wesentlichen gleichmäßiges elektrisches Feld pro Einheitsquerschnitt des bikonkaven, ringförmigen Raums; und
eine gleichmäßige kontinuierliche Abnahme der Stärke des elektrischen Feldes in jeder Richtung weg von dem Mittelabschnitt des bikonkaven, ringförmigen Raums.

14. Behandlungskammer nach einem der Ansprüche 1 bis 12, wobei wenigstens eine der einander gegenüberliegenden Oberflächen der inneren Elektrode (106) bzw. der äußeren Elektrode (104) die Strömungsdynamik des zu behandelnden Fluids in der Behandlungszone steuert.

15. Pasteurisierungsbaugruppe zum Behandeln eines Fluids, die die Behandlungskammer nach einem der Ansprüche 1 bis 14 umfasst.

16. Verfahren zum Pasteurisieren eines Fluids, das **gekennzeichnet ist durch** die folgenden Schritte:
Erzeugen eines elektrischen Feldes zwischen einer inneren Elektrode (106) und einer die innere Elektrode umgebenden äußeren Elektrode (104), wobei einander gegenüberliegende Oberflächen der inneren Elektrode (106) bzw. der äußeren Elektrode (104) eine bikonkave, ringförmige Behandlungszone (111) definieren und wobei die einander gegenüberliegenden Oberflächen der inneren Elektrode (106) bzw. der äußeren Elektrode (104) in der Gesamtbehandlungszone (111) konvex und ununterbrochen sind;
Bewegen des Fluids **durch** die bikonkave, ringförmige Behandlungszone (111), um das Fluid dem elektrischen Feld auszusetzen, um Mikroorganismen in dem Fluid inaktiv zu machen.

17. Verfahren nach Anspruch 16, wobei die Oberfläche der äußeren Elektrode im Wesentlichen toroidförmig ist und die Oberfläche der inneren Elektrode im Wesentlichen ellipsoidförmig ist.

18. Verfahren nach Anspruch 16 oder 17, wobei die Behandlungszone (111) einen Zoneneinlass zum Empfangen von unbehandeltem Fluid, einen Zonenauslass zum Wiedergewinnen von behandeltem Fluid und eine primäre Behandlungszone (112) zum Behandeln des unbehandelten Fluids aufweist, wobei sich die primäre Behandlungszone in dem Mittelabschnitt der Behandlungszone zwischen dem Zoneneinlass (114) und dem Zonenauslass (116) befindet.

19. Verfahren nach Anspruch 18, wobei das elektrische Feld seine größte Stärke in der primären Behandlungszone (112) besitzt.

20. Verfahren nach Anspruch 19, wobei das elektrische Feld in dem Zoneneinlass (114) und in dem Zonenauslass (116) ununterbrochen und im Wesentlichen gleichmäßig pro Einheitsquerschnittsebene ist, wobei seine Stärke von dem Zoneneinlass (114) zu der primären Behandlungszone (112) gleichmäßig zunimmt und von der primären Behandlungszone (112) zu dem Zonenauslass (116) gleichmäßig abnimmt.

21. Verfahren nach Anspruch 20, das ferner die folgenden Schritte umfasst:
Wiedergewinnen des Fluids von einer Fluidquelle und Transportieren des Fluids zu dem Zoneneinlass (114); und
Wiedergewinnen von behandeltem Fluid, nachdem es sich durch den Zonenauslass (116) bewegt hat.

22. Verfahren nach Anspruch 21, wobei eine obere Oberfläche der inneren Elektrode (106) das Fluid von einer Fluidquelle empfängt und es radial durch Überlaufen transportiert, damit es die Oberfläche der inneren Elektrode (106) umgießt, um das Fluid in den Zoneneinlass (114) einzuleiten.

23. Verfahren nach Anspruch 22, wobei die innere Elektrode (106) eine Fluidbohrung aufweist, die längs ihrer Polarachse durch sie verläuft, wobei die Fluidbohrung (526) in der Weise konfiguriert ist, dass die Behandlungszone (111) über einen Fluideinlass (114) in Fluidkommunikation mit der Fluidquelle steht.

24. Verfahren nach Anspruch 23, wobei die innere Elektrode (106) auf ihrer oberen Oberfläche im Wesentlichen eben ist und eine kontinuierliche, gleichmäßige und radiale Kommunikation des Fluids von der Fluidbohrung (526) zu dem Zoneneinlass (114) erleichtert.

25. Verfahren nach Anspruch 22, 23 oder 24, wobei die innere Elektrode (106) auf ihrer oberen Oberfläche eine Vertiefung (452) aufweist, um das Fluid von der Fluidquelle aufzunehmen und das aufgenommene Fluid in einem glatten, stationären und gleichmäßigen Überlaufen radial zu dem Zoneneinlass (114) zu transportieren.

26. Verfahren nach einem der Ansprüche 21 bis 25, das ferner einen Vorbehandlungsschritt umfasst, wobei der Vorbehandlungsschritt dem Einstellen der Temperatur des Fluids auf einen im Voraus definierten Wert dient, bevor das Fluid dem elektrischen Feld ausgesetzt wird.

27. Verfahren nach Anspruch 26, wobei die Temperatur des Fluids durch Bestrahlen des Fluids mit Infrarotstrahlung erhöht wird.

## Revendications

1. Chambre de traitement (500) pour désactiver des micro-organismes dans un liquide, la chambre de traitement comprenant :
une enceinte (502) comprenant une entrée de liquide (505) pour recevoir un liquide à traiter et une sortie de liquide (509) pour pouvoir récupérer un liquide traité ; et
un ensemble électrode (100) à l'intérieur de l'enceinte,
**caractérisée en ce que**
l'ensemble électrode (100) comprend une électrode interne (106) et une électrode externe (104) entourant l'électrode interne ; des surfaces opposées de l'électrode interne (106) et de l'électrode externe (104) définissent une zone de traitement annulaire biconcave (111) ; les surfaces opposées de l'électrode interne (106) et de l'électrode externe (104) sont convexes et continues dans toute la zone de traitement.

2. Chambre de traitement (500) selon la revendication 1, dans laquelle l'ensemble électrode (100) est configuré pour recevoir une impulsion de tension qui génère un champ électrique entre l'électrode interne (106) et l'électrode externe (104), l'intensité du champ électrique diminuant en une diminution continue régulière de l'intensité du champ électrique dans n'importe quelle direction en s'éloignant d'une section centrale de la zone de traitement (111) quand l'impulsion de tension est appliquée aux électrodes (104, 106).

3. Chambre de traitement (500) selon la revendication 1 ou 2, dans laquelle la surface convexe de l'électrode externe (104) est sensiblement toroïdale et la section convexe de l'électrode interne (106) est sensiblement ellipsoïdale.

4. Chambre de traitement (500) selon la revendication 3, dans laquelle la surface convexe de l'électrode interne (106) est sensiblement sphérique.

5. Chambre de traitement (500) selon la revendication 1 ou 2, dans laquelle la surface convexe de l'électrode externe (104) comprend une pluralité de surfaces sensiblement ellipsoïdales adjacentes et la surface convexe de l'électrode interne (106) est sensiblement ellipsoïdale.

6. Chambre de traitement (500) selon la revendication 5, dans laquelle la surface convexe de l'électrode interne (106) est sensiblement sphérique.

7. Chambre de traitement de liquide selon l'une quelconque des revendications précédentes, dans laquelle la zone de traitement (111) comprend une entrée de zone (114) pour recevoir un liquide non traité, une sortie de zone (116) pour délivrer un liquide traité, et une zone de traitement principale (112) pour traiter le liquide non traité, la zone de traitement principale (112) étant située dans la section centrale de la zone de traitement (111), entre l'entrée de zone (114) et la sortie de zone (116).

8. Chambre de traitement (500) selon la revendication 7, dans laquelle une surface supérieure de l'électrode interne (106) reçoit le liquide depuis une source de liquide et l'achemine radialement par débordement pour inonder la surface de l'électrode interne afin d'introduire le liquide dans l'entrée de zone (114) et la zone de traitement principale (112).

9. Chambre de traitement de liquide (500) selon la revendication 8, dans laquelle l'intensité du champ électrique augmente progressivement depuis l'entrée de zone (114) vers la zone de traitement principale (112) et diminue ensuite progressivement depuis la zone de traitement principale (112) vers la sortie de zone (116).

10. Chambre de traitement de liquide (500) selon la revendication 8, dans laquelle l'électrode interne (106) comporte un alésage pour liquide (526) s'étendant à travers celle-ci le long de son axe polaire, l'alésage pour liquide (526) étant configuré de telle sorte que la zone de traitement (111) est en communication fluidique avec l'entrée de liquide.

11. Chambre de traitement de liquide (500) selon la revendication 10, dans laquelle l'électrode interne (106) est sensiblement plane sur sa surface supérieure et agencée pour faciliter une communication continue, uniforme et radiale du liquide depuis l'alésage pour liquide (526) jusqu'à l'entrée de zone (114).

12. Chambre de traitement de liquide (500) selon la revendication 8, 9, 10 ou 11, dans laquelle l'électrode interne (106) comprend un creux (452) sur sa surface supérieure pour recevoir le liquide depuis la source de liquide.

13. Chambre de traitement (500) selon l'une quelconque des revendications 1 à 12, dans laquelle sont simultanément produits par l'application d'une impulsion de tension aux électrodes interne et externe (104, 106) :
un champ électrique le plus intense généré par l'électrode interne (106) et l'électrode externe (104) dans une section centrale de l'espace annulaire biconcave ;
un champ électrique sensiblement uniforme par unité de section transversale de l'espace annulaire biconcave ; et
une diminution continue régulière de l'intensité du champ électrique dans n'importe quelle direction en s'éloignant de la section centrale de l'espace annulaire biconcave.

14. Chambre de traitement selon l'une quelconque des revendications 1 à 12, dans laquelle l'une au moins des surfaces opposées de l'électrode interne (106) et de l'électrode externe (104) contrôle la dynamique d'écoulement du liquide à traiter à l'intérieur de la zone de traitement.

15. Trousse de pasteurisation pour traiter un liquide comprenant la chambre de traitement de l'une quelconque des revendications 1 à 14.

16. Procédé de pasteurisation d'un liquide **caractérisé par** les étapes consistant à :
générer un champ électrique entre une électrode interne (106) et une électrode externe (104) entourant l'électrode interne, des surfaces opposées de l'électrode interne (106) et de l'électrode externe (104) définissant une zone de traitement annulaire biconcave (111), les surfaces opposées de l'électrode interne (106) et de l'électrode externe (104) étant convexes et continues dans toute la zone de traitement (111) ;
faire passer le liquide à travers la zone de traitement annulaire biconcave (111) pour exposer le liquide au champ électrique afin d'inactiver des micro-organismes dans le liquide.

17. Procédé selon la revendication 16, dans lequel la surface de l'électrode externe est sensiblement toroïdale et la surface de l'électrode interne est sensiblement ellipsoïdale.

18. Procédé selon la revendication 16 ou 17, dans lequel la zone de traitement (111) comprend une entrée de zone pour recevoir un liquide non traité, une sortie de zone pour récupérer un liquide traité et une zone de traitement principale (112) pour traiter le liquide non traité, la zone de traitement principale étant située dans la section centrale de la zone de traitement entre l'entrée de zone (114) et la sortie de zone (116).

19. Procédé selon la revendication 18, dans lequel le champ électrique a son intensité la plus forte à l'intérieur de la zone de traitement principale (112).

20. Procédé selon la revendication 19, dans lequel le champ électrique est continu et sensiblement uniforme par unité de plan transversal dans l'entrée de zone (114) et la sortie de zone (116), son intensité augmentant régulièrement depuis l'entrée de zone (114) vers la zone de traitement principale (112) et diminuant régulièrement depuis la zone de traitement principale (112) jusqu'à la sortie de zone (116).

21. Procédé selon la revendication 20 comprenant en outre les étapes consistant à :
récupérer le liquide depuis une source de liquide et l'acheminer jusqu'à l'entrée de zone (114) ; et
récupérer un liquide traité après qu'il est passé par la sortie de zone (116).

22. Procédé selon la revendication 21, dans lequel une surface supérieure de l'électrode interne (106) reçoit le liquide depuis une source de liquide et l'achemine radialement par débordement pour inonder la surface de l'électrode interne (106) afin d'introduire le liquide dans l'entrée de zone (114).

23. Procédé selon la revendication 22, dans lequel l'électrode interne (106) comporte un alésage pour liquide s'étendant à travers celle-ci le long de son axe polaire, l'alésage pour liquide (526) étant configuré de telle sorte que la zone de traitement (111) est en communication fluidique avec la source de liquide par le biais d'une entrée de liquide (114).

24. Procédé selon la revendication 23, dans lequel l'électrode interne (106) est sensiblement plane sur sa surface supérieure et facilite une communication continue, uniforme et radiale du liquide depuis l'alésage pour liquide (526) jusqu'à l'entrée de zone (114).

25. Procédé selon la revendication 22, 23 ou 24, dans lequel l'électrode interne (106) comprend un creux (452) sur sa surface supérieure pour recevoir le liquide depuis la source de liquide et acheminer le liquide reçu en un débordement régulier, constant et uniforme radialement vers l'entrée de zone (114).

26. Procédé selon l'une quelconque des revendications 21 à 25 comprenant en outre une étape de prétraitement, l'étape de prétraitement étant destinée à ajuster la température du liquide à un niveau prédéfini avant d'exposer le liquide au champ électrique.

27. Procédé selon la revendication 26, dans lequel la température du liquide est augmentée en irradiant le liquide avec un rayonnement infrarouge.
